# EUROPÄISCHE PATENTSCHRIFT

(11) **EP 0 910 660 B1**
(45) Veröffentlichungstag und Bekanntmachung des Hinweises auf die Patenterteilung: **22.01.2003**
(21) Anmeldenummer: 97923936.5
(22) Anmeldetag: 20.05.1997
(51) Int. Cl.: C12P 41/00, C12P 13/02, C07C 233/64

(54) **VERFAHREN ZUR HERSTELLUNG VON OPTISCH AKTIVEN AMINEN**
PROCESS FOR PRODUCING OPTICALLY ACTIVE AMINES
PROCEDE DE PRODUCTION D'AMINES OPTIQUEMENT ACTIVES

(30) Priorität: 30.05.1996 DE 19621686
(43) Veröffentlichungstag der Anmeldung: 28.04.1999
(73) Patentinhaber: Bayer CropScience AG, 40789 Monheim (DE)
(72) Erfinder: STELZER, Uwe, D-51399 Burscheid (DE)
(86) Internationale Anmeldenummer: EP9702547
(87) Internationale Veröffentlichungsnummer: WO97046698

(56) Entgegenhaltungen:
- EP-A- 0 399 589
- WO-A-91/19002
- DE-A- 4 332 738
- GB-A- 2 246 774
- CHEMICAL ABSTRACTS, vol. 115, no. 25, 23.Dezember 1991 Columbus, Ohio, US; abstract no. 278181, ITO, ISAMU ET AL: "Enzymic resolution of racemic amines" XP002041026 & JP 03 191 797 A (FUJI PHOTO FILM CO., LTD., JAPAN)
- CHEMICAL ABSTRACTS, vol. 115, no. 19, 11.November 1991 Columbus, Ohio, US; abstract no. 206019, KITAGUCHI, HIROSHI ET AL: "Enzymic resolution of racemic amines. Crucial role of the solvent" XP002041027 & ANN. N. Y. ACAD. SCI. (1990), 613(ENZYME ENG. 10), 656-8 CODEN: ANYAA9;ISSN: 0077-8923,
- VOERDE, CARIN ET AL: "Resolution of 2-methylalkanoic esters: enantioselective aminolysis by (R)-1-phenylethylamine of ethyl 2-methyloctanoate catalyzed by lipase B from Candida antarctica" TETRAHEDRON: ASYMMETRY (1996), 7(5), 1507-1513 CODEN: TASYE3;ISSN: 0957-4166, XP002041024
- QUIROS, MARGARITA ET AL: "Lipase -catalyzed synthesis of optically active amides in organic media" TETRAHEDRON: ASYMMETRY (1993), 4(6), 1105-12 CODEN: TASYE3;ISSN: 0957-4166, XP002041025
- DATABASE WPI Section Ch, Week 9732 Derwent Publications Ltd., London, GB; Class B05, AN 97-344901 XP002041028 & JP 09 140 389 A (KAWAKEN FINE CHEM CO LTD) , 3.Juni 1997

## Beschreibung

Die vorliegende Erfindung betrifft ein neues Verfahren zur Herstellung von bekannten, optisch aktiven Aminen, die als Zwischenprodukte zur Herstellung von Pharmazeutika und Pflanzenschutzmitteln verwendet werden können. Die Erfindung betrifft außerdem neue optisch aktive, acylierte Amine.

Aus der DE-A 4 332 738 ist bereits bekannt, daß sich optisch aktive, primäre und sekundäre Amine herstellen lassen, indem man zunächst racemisches Amin in Gegenwart einer Hydrolase mit einem Ester, der im Säureteil in Nachbarschaft des Carbonylkohlenstoffatoms ein elektronenreiches Heteroatom aufweist, enantioselektiv acyliert, dann das entstehende Gemisch aus optisch aktivem (S)-Amin und optisch aktivem acylierten (R)-Amin (= Amid) trennt, dadurch das (S)-Amin erhält und gegebenenfalls aus dem acylierten (R)-Amin durch Amid-Spaltung das andere Enantiomere gewinnt. Als Hydrolasen kommen dabei Lipasen aus Pseudomonas, z.B. Amano P, oder aus Pseudomonas spec. DSM 8246 in Frage. Der optische Reinheitsgrad der anfallenden Enantiomeren ist sehr hoch. Nachteilig an diesem Verfahren ist aber, daß bei der enzymatischen Acylierung recht lange Reaktionszeiten erforderlich sind und in stark verdünnter Lösung gearbeitet wird. Das verbleibende (S)-Enantiomer wird erst nach relativ langen Reaktionszeiten in ausreichend hoher optischer Ausbeute erhalten. Die erzielbaren Raum-Zeit-Ausbeuten lassen daher für praktische Zwecke zu wünschen übrig. Ungünstig ist auch, daß in Bezug auf das Substrat verhältnismäßig hohe Mengen an Enzym erforderlich sind. Im übrigen weist das Enzym eine sehr hohe Aktivität auf, so daß ein erheblicher Aufwand für die Reinigung, die Konzentrierung und die Aufarbeitung notwendig ist. Außerdem ist ein relativ teurer Acylierungsbaustein erforderlich.

Weiterhin geht aus Chimica 48, 570 (1994) hervor, daß racemische Amine mit Essigsäureethylester in Gegenwart von Lipase aus Candida antarctica enantioselektiv zu Gemischen aus (S)-Amin und acetyliertem (R)-Amin (= Amid) reagieren, aus denen (S)-Amin und acetyliertes (R)-Amin isoliert werden können, wobei das acetylierte (R)-Amin durch anschließende Amid-Spaltung freigesetzt werden kann. Ungünstig an dieser Methode ist, daß wiederum recht lange Reaktionszeiten benötigt werden und außerdem die Ausbeuten nicht immer befriedigend sind. Weiterhin ist auch hierbei das Verhältnis von Enzym zu Substrat so unvorteilhaft, daß eine wirtschaftliche Nutzung des Verfahrens kaum möglich ist.

Aus JP-A 03-191 797, Chem. Abstr. 115, 278 181, Ann. N. Y. Acad. Sci. 613, 656-659 (1990) und WO-A 91-91 002 geht hervor, dass sich optisch aktive Amine herstellen lassen, indem man racemische Amine mit Estern in Gegenwart eines Biokatalysators behandelt. Keiner der Ester enthält jedoch eine aktivierende Gruppe. Außerdem wird Lipase aus Candida antarctica nicht als möglicher Biokatalysator genannt.

Es wurde nun gefunden, daß man optisch aktive Amine der Formel 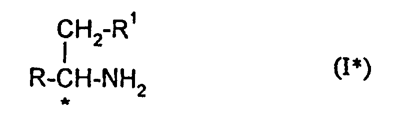 in welcher
- R: für einen Rest der Formel

-(CH₂)ₘ-R²

steht, worin
R² für gegebenenfalls substituiertes Phenyl der Formel 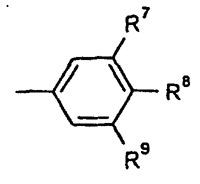 steht, wobei
R⁷, R⁸ und R⁹ unabhängig voneinander für Wasserstoff, Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Cyano, Dialkylamino mit 1 bis 4 Kohlenstoffatomen in jeder Alkylgruppe, Nitro, Phenyl, Phenoxy oder Benzyl stehen, und
m für die Zahlen 0, 1, 2 oder 3 steht,
- R¹: für Wasserstoff steht,
erhält, indem man
a) in einer ersten Stufe racemische Amine der Formel 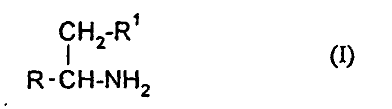 in welcher
   R und R¹ die oben angegebenen Bedeutungen haben,
   mit Estern der Formel 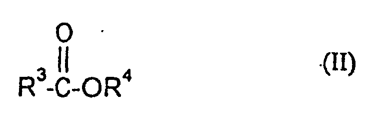 in welcher
   - R³: geradkettiges Halogenalkyl mit 1 bis 4 Kohlenstoffatomen und 1 bis 3 Fluor- und/oder Chloratomen oder für einen Rest der Formel -CH₂-C≡N steht,
   oder
   - R³: für einen Rest der Formel -CH₂-COOR⁶ steht, worin
   R⁶ für Methyl, Ethyl, n-Propyl oder n-Butyl steht, und
   - R⁴: für geradkettiges Alkyl mit 1 bis 8 Kohlenstoffatomen steht, in Gegenwart von Lipase ans Candida antarctica und in Gegenwart eines Verdünnungsmittels umsetzt,
b) in einer zweiten Stufe das erhaltene Gemisch aus (S)-Amin der Formel 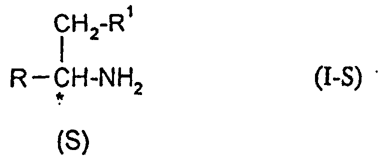 in welcher
   R und R¹ die oben angegebenen Bedeutungen haben,
   und acyliertem (R)-Amin der Formel 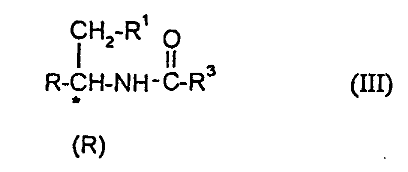 in welcher
   R, R¹ und R³ die oben angegebenen Bedeutungen haben,
   trennt und
c) gegebenenfalls in einer dritten Stufe aus acyliertem (R)-Amin der Formel (III) durch Behandlung mit Säure oder Base, gegebenenfalls in Gegenwart eines Verdünnungsmittels (R)-Amin der Formel 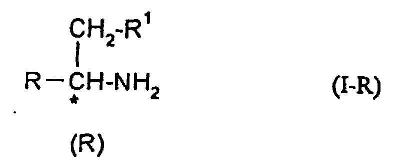 in welcher
   R und R¹ die oben angegebenen Bedeutungen haben,
freisetzt.

Unter (R)-Aminen sind diejenigen optisch aktiven Verbindungen der Formel (I) zu verstehen, die am asymmetrisch substituierten Kohlenstoffatom die (R)-Konfiguration aufweisen. Entsprechend sind unter (S)-Aminen diejenigen optisch aktiven Verbindungen der Formel (I) zu verstehen, die am Chiralitätszentrum die (S)-Konfiguration aufweisen. In den Formeln ist das asymmetrisch substituierte Kohlenstoffatom jeweils durch (*) gekennzeichnet.

Es ist als äußerst überraschend zu bezeichnen, daß sich optisch aktive Amine der Formel (I*) nach dem erfindungsgemäßen Verfahren in hoher Ausbeute und sehr guter optischer Reinheit herstellen lassen. Aufgrund des bekannten Standes der Technik konnte nämlich nicht damit gerechnet werden, daß eine enantioselektive Amin-Synthese auch mit solchen Estern möglich ist, die im Säureteil in Nachbarschaft zum Carbonyl-Kohlenstoffatom kein elektronenreiches Heteroatom aufweisen. Außerdem war nicht zu erwarten, daß sich nach dem erfindungsgemäßen Verfahren bessere Ergebnisse erzielen lassen als durch entsprechende Umsetzung unter Verwendung von Essigsäureethylester als Acylierungskomponente.

Das erfindungsgemäße Verfahren weist eine Reihe von Vorteilen auf. So ermöglicht es die Herstellung einer Vielzahl von optisch aktiven Aminen in hoher Ausbeute und hervorragender optischer Reinheit. Günstig ist auch, daß bei relativ hoher Substrat-Konzentration gearbeitet werden kann und die Reaktionszeiten kurz sind. Dadurch lassen sich Raum-Zeit-Ausbeuten erzielen, die auch für praktische Zwecke befriedigend sind. Weiterhin handelt es sich bei den Acylierungskomponenten um preiswerte und einfach zugängliche Stoffe. Von Vorteil ist auch, daß der benötigte Biokatalysator in größerer Menge zur Verfügung steht und auch bei erhöhter Temperatur stabil ist. Dabei wird der Biokatalysator bezüglich der

Enzym-Menge im Verhältnis zum Substrat in relativ geringer Menge und niedriger Enzymaktivität eingesetzt. Schließlich bereitet auch die Durchführung der Umsetzung und die Isolierung der gewünschten Substanzen, und zwar sowohl der (S)- als auch der (R)-Amine, keinerlei Schwierigkeiten.

Setzt man racemisches 1-(4-Chlor-phenyl)-ethylamin in Gegenwart von Lipase aus Candida antarctica mit Essigsäure-n-butylester um, trennt die entstehenden Komponenten und behandelt das (R)-Enantiomere des Essigsäure-1-(4-chlor-phenyl)-ethylamids mit Salzsäure, so kann der Verlauf des erfindungsgemäßen Verfahrens durch das folgende Formelschema veranschaulicht werden.

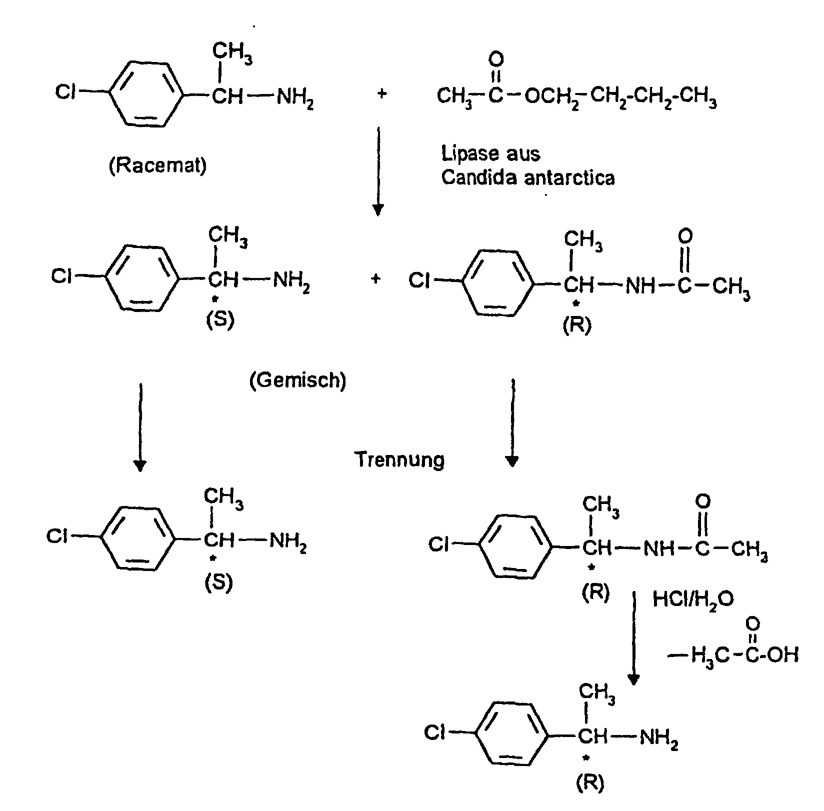

Die bei der Durchführung des erfindungsgemäßen Verfahrens als Ausgangsstoffe benötigten racemischen Amine sind durch die Formel (I) allgemein definiert.

Besonders bevorzugt sind Amine der Formel (I), in denen
- R: für einen Rest der Formel

-(CH₂)ₘ-R²

steht, worin
R² bevorzugt für gegebenenfalls substituiertes Phenyl der Formel 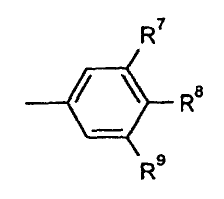 steht, wobei
R⁷, R⁸ und R⁹ unabhängig voneinander für Wasserstoff, Fluor, Chlor, Brom, Methyl, Ethyl, n-Propyl, Isopropyl, n-Butyl, iso-Butyl, sek.-Butyl, Methoxy, Ethoxy, Methylthio, Trichlormethyl, Trifluormethyl, Difluormethyl, Trifluormethoxy, Difluorchlormethoxy, Difluormethoxy, Cyano, Dimethylamino, Diethylamino, Nitro, Phenyl, Phenoxy oder Benzyl stehen, und
m für die Zahlen 0, 1 oder 2 steht, und
- R¹: für Wasserstoff steht.

Als Beispiele für racemische Amine der Formel (I) seien die Verbindungen der folgenden Formeln genannt:

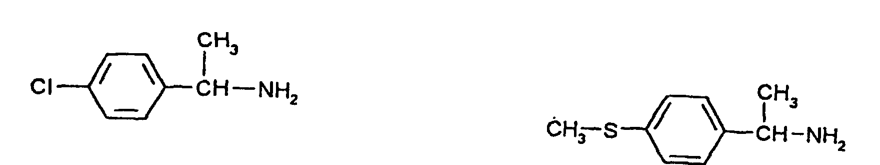

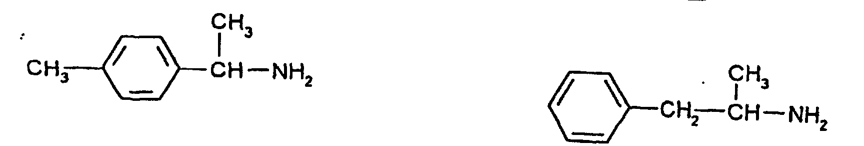

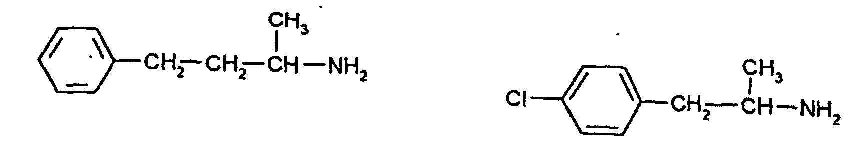

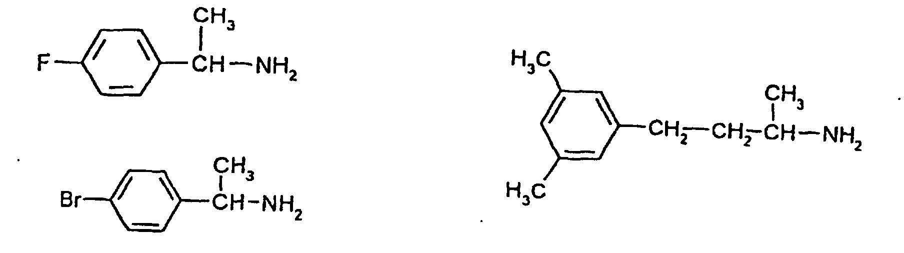

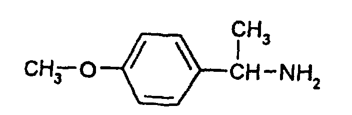

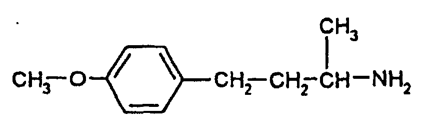

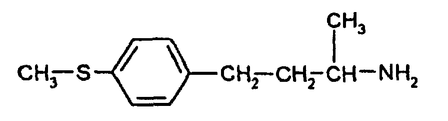

Die racemischen Amine der Formel (I) sind bekannt oder lassen sich nach bekannten Methoden herstellen.

Die bei der Durchführung der ersten Stufe des erfindungsgemäßen Verfahrens als Reaktionskomponenten benötigten Ester sind durch die Formel (II) allgemein definiert.

Besonders bevorzugt sind Ester der Formel (II), in denen
- R³: für Chlormethyl, Fluormethyl, Trifluormethyl, 2-Chlorethyt oder für einen Rest der Formel -CH₂-C≡N steht,
oder
R³ für einen Rest der Formel -CH₂-COOR⁶ steht, worin
R⁶ für Methyl, Ethyl, n-Propyl oder n-Butyl steht, und
- R⁴: für Methyl, Ethyl, n-Propyl oder n-Butyl steht.

Als Beispiele für Ester der Formel (II) seien die Verbindungen der folgenden Formeln genannt.

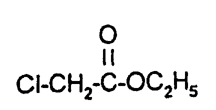

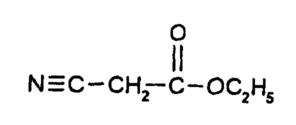

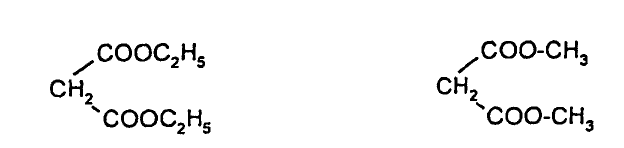

Die Ester der Formel (II) sind bekannt oder lassen sich nach bekannten Methoden herstellen.

Als Hydrolase dient bei der Durchführung der ersten Stufe des erfindungsgemäßen Verfahrens Lipase aus Candida antarctica (=Novozym 435®).

Die genannte Substanz ist bekannt. So wird die Herstellung von Lipase aus Candida antarchica in der Literatur beschrieben (vgl. Ind. J. Chem. 32B, 76-80 (1993) und EP-A 0 287 634). Lipase aus Candida antarctica ist unter der Bezeichnung Novozym 435® kommerziell erhältlich.

Die Hydrolase kann entweder nativ oder in modifizierter Form, z.B. mikroverkapselt oder an anorganische oder organische Trägermaterialien gebunden, eingesetzt werden. Als Trägermaterialien kommen dabei z.B. Celite, Lewatit, Zeolithe, Polysaccharide, Polyamide und Polystyrolharze in Frage.

Als Verdünnungsmittel kommen bei der Durchführung der ersten Stufe des erfindungsgemäßen Verfahrens alle für derartige Umsetzungen üblichen organischen Solventien in Betracht. Vorzugsweise verwendbar sind Ether, wie Methyl-tert.-butyl-ether, Dimethoxyethan, oder tert.-Amyl-methyl-ether, ferner aliphatische oder aromatische Kohlenwasserstoffe, wie Hexan, Cyclohexan oder Toluol, außerdem Nitrile, wie Acetonitril oder Butyronitril, weiterhin Alkohole, wie tert.-Butanol oder 3-Methyl-3-pentanol, und schließlich auch die zur Acylierung eingesetzten Ester.

Die Temperaturen können bei der Durchführung der ersten Stufe des erfindungsgemäßen Verfahrens innerhalb eines bestimmten Bereiches variiert werden. Im allgemeinen arbeitet man bei Temperaturen zwischen 0°C und 80°C, vorzugsweise zwischen 10°C und 60°C.

Bei der Durchführung der ersten Stufe des erfindungsgemäßen Verfahrens arbeitet man im allgemeinen unter Atmosphärendruck, gegebenenfalls unter Inertgas, wie Stickstoff oder Argon.

Bei der Durchführung der ersten Stufe des erfindungsgemäßen Verfahrens setzt man auf 1 Mol an racemischem Amin der Formel (I) im allgemeinen 0,6 bis 10 Mol, vorzugsweise 1 bis 3 Mol an Ester der Formel (II) ein. Die Menge an Hydrolase kann ebenfalls innerhalb eines bestimmten Bereiches variiert werden. Im allgemeinen setzt man 1 bis 10 Gew.-% an immobilisierter Hydrolase, bezogen auf racemisches Amin ein, was einer Aktivität von 10 000 bis 112 000 Units Hydrolase pro Mol an racemischem Amin entspricht. Im einzelnen verfährt man bei der Durchführung der ersten Stufe des erfindungsgemäßen Verfahrens in der Weise, daß man die Komponenten in beliebiger Reihenfolge zusammengibt und das entstehende Gemisch bis zum Erreichen des gewünschten Umsatzes bei der jeweiligen Reaktionstemperatur rührt. Zur Beendigung der Reaktion geht man im allgemeinen so vor, daß man den Biokatalysator durch Filtration abtrennt.

Das in der ersten Stufe des erfindungsgemäßen Verfahrens erhaltene Gemisch wird in der zweiten Stufe nach üblichen Methoden aufgearbeitet. Im allgemeinen isoliert man die gewünschten Komponenten durch Destillation, fraktionierte Kristallisation, Säure-Base-Lösungsmittel-Extraktion oder auf anderem Wege. So kann man zum Beispiel in der Weise verfahren, daß man das Reaktionsgemisch einer fraktionierten Destillation unterwirft. Es ist auch möglich, so vorzugehen, daß man das Reaktionsgemisch einengt, den verbleibenden Rückstand in einem mit Wasser nur wenig mischbaren, organischen Solvens aufnimmt, die entstehende Lösung mit Wasser und Mineralsäure behandelt und die Phasen trennt. Durch Einengen der organischen Phase erhält man das acylierte (R)-Amin. Aus der wäßrigen Phase läßt sich das (5)-Amin isolieren, indem man zunächst mit Base behandelt, dann mit einem mit Wasser nur wenig mischbaren organischen Solvens extrahiert, die vereinigten organischen Phasen trocknet und einengt. - Gegebenenfalls kann eine weitere Reinigung der isolierten Produkte, z.B. durch Chromatographie oder Destillation, vorgenommen werden.

Die acylierten (R)-Amine der Formel 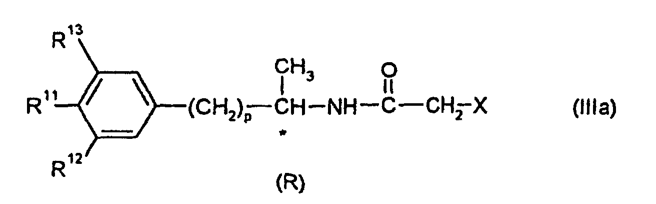 in welcher
- R¹³ und R¹²: für Methyl stehen,
- R¹¹: für Wasserstoff steht,
- p: für die Zahl 2 steht und
- X: für Chlor oder Cyano steht,
oder
- R¹³, R¹¹ und R¹²: für Wasserstoff stehen,
- p: für die Zahlen 1 oder 2 steht und
- X: für Chlor oder Cyano steht,
oder
- R¹¹: für Fluor, Chlor, Brom, Methyl, Methoxy oder Methylthio steht,
- R¹³ und R¹²: für Wasserstoff stehen,
- p: für die Zahlen 0, 1 oder 2 steht und
- X: für Chlor oder Cyano steht,
sind neu.

Als Beispiele für acylierte (R)-Amine der Formel (IIIa) seien die Verbindungen der folgenden Formeln genannt:

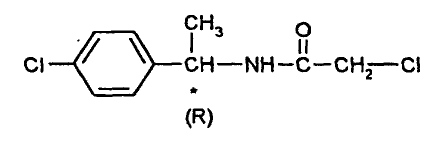

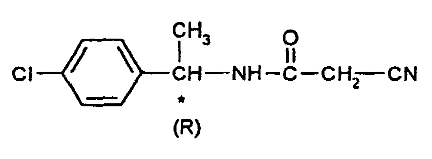

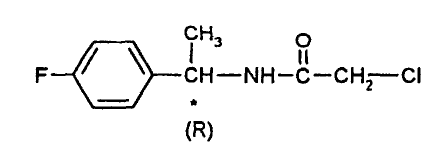

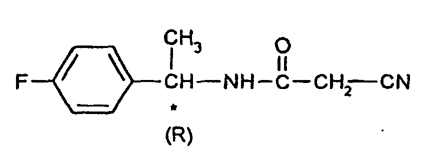

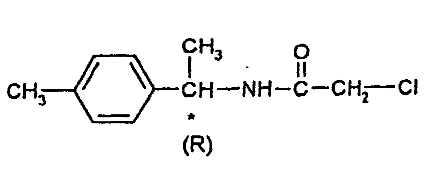

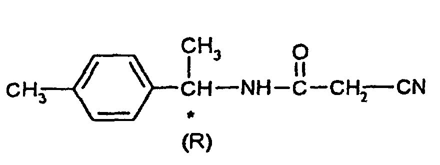

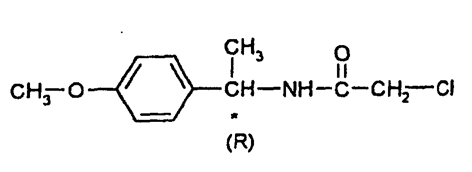

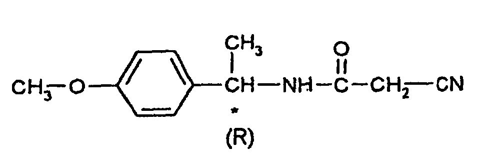

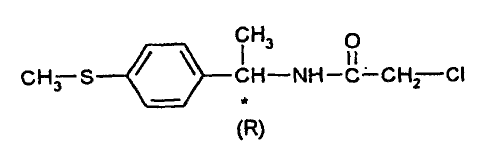

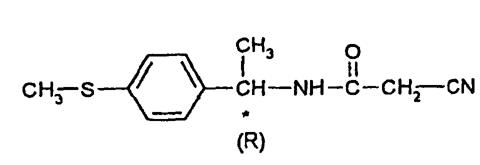

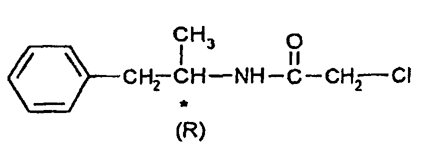

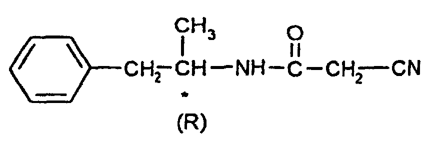

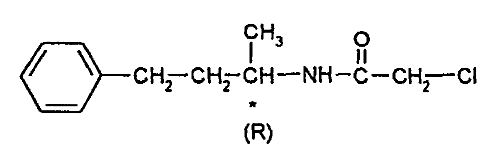

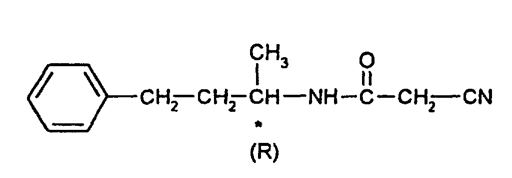

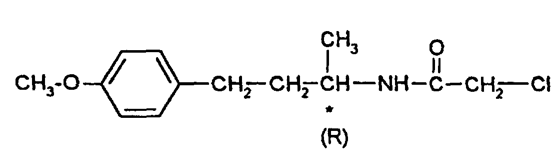

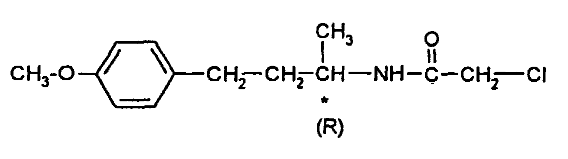

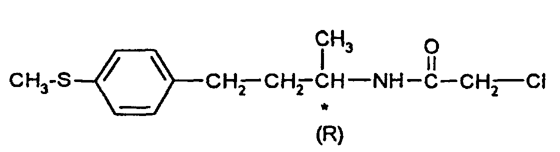

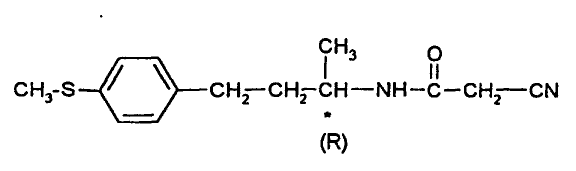

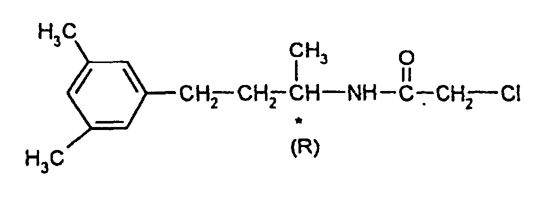

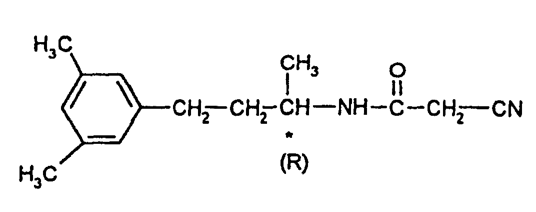

Als Säuren kommen bei der Durchführung der dritten Stufe des erfindungsgemäßen Verfahrens alle üblichen starken Säuren in Betracht. Vorzugsweise verwendbar sind Mineralsäuren, wie Schwefelsäure oder Salzsäure.

Als Basen kommen bei der Durchführung der dritten Stufe des erfindungsgemäßen Verfahrens alle üblichen starken Basen in Betracht. Vorzugsweise verwendbar sind anorganische Basen, wie Natrium- oder Kaliumhydroxid.

Als Vedünnungsmittel kommen bei der Durchführung der dritten Stufe des erfindungsgemäßen Verfahrens alle für derartige Umsetzungen üblichen organischen Solventien sowie Wasser in Betracht. Vorzugsweise verwendbar sind Wasser oder Mischungen aus Wasser und organischen Solventien, wobei Gemische aus Wasser und Toluol beispielhaft genannt seien.

Die Temperaturen können bei der Durchführung der dritten Stufe des erfindungsgemäßen Verfahrens innerhalb eines größeren Bereiches variiert werden. Im allgemeinen arbeitet man bei Temperaturen zwischen 20 und 180°C, vorzugsweise zwischen 30 und 150°C.

Bei der Durchführung der dritten Stufe des erfindungsgemäßen Verfahrens arbeitet man im allgemeinen unter Atmosphärendruck. Es ist aber auch möglich, unter erhöhtem oder vermindertem Druck zu arbeiten.

Bei der Durchführung der dritten Stufe des erfindungsgemäßen Verfahrens setzt man auf 1 Mol an acyliertem (R)-Amin der Formel (III) im allgemeinen 1 bis 5 Äquivalente oder auch einen größeren Überschuß an Säure oder Base ein. Die Aufarbeitung erfolgt nach üblichen Methoden. Im allgemeinen geht man so vor, daß man das Reaktionsgemisch nach beendeter Spaltung und Neutralisation mit einem mit Wasser nur wenig mischbaren, organischen Solvens extrahiert, die vereinigten organischen Phasen trocknet und einengt. Das dabei erhaltene Produkt kann gegebenenfalls nach üblichen Methoden von eventuell noch vorhandenen Verunreinigungen befreit werden.

Die nach dem erfindungsgemäßen Verfahren herstellbaren Amine der Formel (I*) sind wertvolle Zwischenprodukte zur Herstellung von Pharmazeutika oder von Wirkstoffen mit insektiziden, fungiziden oder herbiziden Eigenschaften (vgl. EP-A 0 519 211, EP-A 0 453 137, EP-A 0 283 879, EP-A 0 264 217 und EP-A 0 341 475). So erhält man z.B. die fungizid wirksame Verbindung der Formel 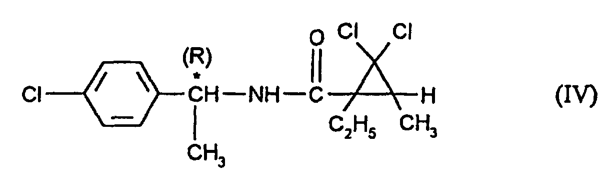 indem man das (R)-1-(4-Chlor-phenyl)-ethylamin der Formel 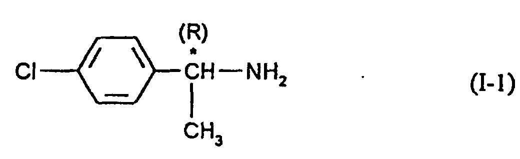 mit 2,2-Dichlor-1-ethyl-3-methyl-1-cyclopropancarbonsäurechlorid der Formel 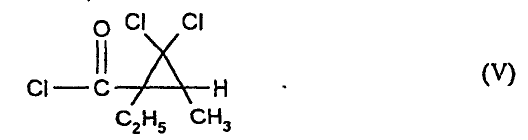 in Gegenwart eines Säurebindemittels und in Gegenwart eines inerten organischen Verdünnungsmittels umsetzt.

Die Durchführung des erfindungsgemäßen Verfahrens wird durch die folgenden Beispiele veranschaulicht.

### Herstellungsbeispiele

### Beispiel 1

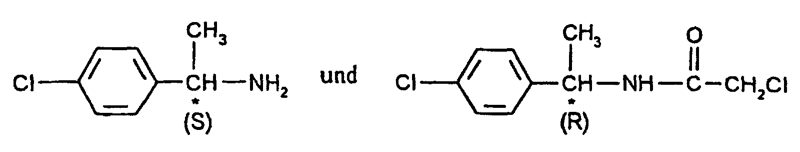

### 1. Stufe

Eine Lösung von 4,67 g (0,03 mol) an racemischem 1-(4-Chlor-phenyl)-ethylamin in 40 ml Methyl-tert.-butyl-ether wird bei Raumtemperatur unter Rühren nacheinander mit 5,5 g (0,045 mol) Chloressigsäureethylester und 0,4 g Novozym 435® (= immobilisierte Lipase aus Candida antarctica; 7300 U/g) versetzt. Man rührt weiter bei Raumtemperatur und kontrolliert das Fortschreiten der Reaktion, indem man Proben gaschromatographisch untersucht. Nach einer Stunde ist ein Umsatz von 51 % erreicht. Zu diesem Zeitpunkt wird die Reaktion abgebrochen, indem man das Enzym abfiltriert. In dem verbleibenden Filtrat weist das (S)-Enantiomere des 1-(4-Chlor-phenyl)-ethylamins einen ee-Wert von 89,1 % auf, während das (R)-Enantiomere des Chloressigsäure-1-(4-chlor-phenyl)-ethylamids mit einem ee-Wert von 95,5 % anfällt.

### 2. Stufe

Das nach dem Abfiltrieren des Enzyms verbleibende Filtrat wird unter vermindertem Druck eingeengt. Der anfallende Rückstand wird mit 40 ml einer 5 %igen, wäßrigen Salzsäure versetzt und 2 Stunden bei Raumtemperatur gerührt. Das Gemisch wird dreimal mit Methylenchlorid extrahiert. Die vereinigten organischen Phasen werden über Natriumsulfat getrocknet und danach unter vermindertem Druck eingeengt. Man erhält auf diese Weise 3,08 g eines Produktes, das gemäß gaschromatographischer Analyse zu 95,7 % aus dem (R)-Enantiomeren des Chloressigsäure-1-(4-chlor-phenyl)-ethylamids besteht. Der ee-Wert beträgt 97,5 %.

### Beispiel 2

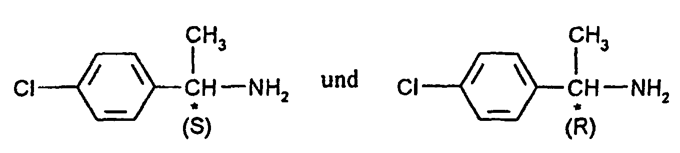

### 1. Stufe

Eine Lösung von 6,3 g (0,04 mol) an racemischem 1-(4-Chlor-phenyl)-ethylamin in 45 ml Methyl-tert.-butyl-ether wird bei 35°C unter Rühren nacheinander mit 4,9 g (0,04 mol) Chloressigsäureethylester und 0,5 g Novozym 435® (= immobilisierte Lipase aus Candida antarctica; 7300 U/g) versetzt. Man rührt weiter bei 35°C und kontrolliert das Fortschreiten der Reaktion, indem man Proben gaschromatographisch untersucht. Nach 4 Stunden ist ein Umsatz von 54 % erreicht. Zu diesem Zeitpunkt wird die Reaktion abgebrochen, indem man das Enzym abfiltriert. In dem verbleibenden Filtrat weist das (S)-Enantiomere des 1-(4-Chlor-phenyl)-ethylamins einen ee-Wert von 96,2 % auf, während das (R)-Enantiomere des Chloressigsäure-1-(4-chlor-phenyl)-ethylamids mit einem ee-Wert von 95,1 % anfällt.

### 2. Stufe

Das nach dem Abfiltrieren des Enzyms verbleibende Filtrat wird unter vermindertem Druck eingeengt. Der anfallende Rückstand wird mit 40 ml einer 5 %igen, wäßrigen Salzsäure versetzt und 0,5 Stunden bei Raumtemperatur gerührt. Das Gemisch wird dreimal mit Methylenchlorid extrahiert. Die vereinigten organischen Phasen werden über Natriumsulfat getrocknet und danach unter vermindertem Druck eingeengt. Der Rückstand wird mit 40 ml 15 %iger, wäßriger Salzsäure versetzt und 3 Stunden unter Rückfluß erhitzt. Danach wird das Reaktionsgemisch auf Raumtemperatur abgekühlt, mit wäßriger Natronlauge basisch gestellt und mehrfach mit Methylenchlorid extrahiert. Die vereinigten organischen Phasen werden über Natriumsulfat getrocknet und unter vermindertem Druck eingeengt. Man erhält auf diese Weise 4,23 g eines Produktes, das gemäß gaschromatographischer Analyse zu 97 % aus dem (R)-Enantiomeren des 1-(4-Chlor-phenyl)-ethylamins besteht. Der ee-Wert beträgt 95,1 %.

### 3. Stufe

Die wäßrige Phase, die nach der oben beschriebenen Behandlung mit 5 %iger wäßriger Salzsäure anfällt, wird durch Zugabe von wäßriger Natronlauge alkalisch gestellt und mehrfach mit Methylenchlorid extrahiert. Die vereinigten organischen Phasen werden über Natriumsulfat getrocknet und dann unter vermindertem Druck eingeengt. Man erhält auf diese Weise 2,38 g eines Produktes, das gemäß gaschromatographischer Analyse zu 93 % aus dem (S)-Enantiomeren des 1-(4-Chlorphenyl)-ethylamins besteht. Der ee-Wert beträgt 96,2 %.

### Beispiel 3

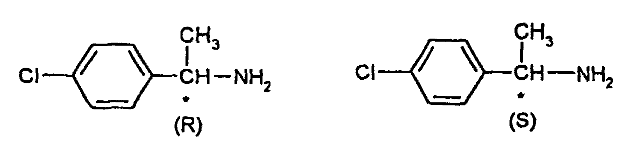

und

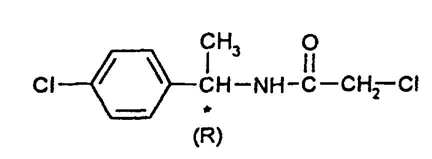

### 1. Stufe

Eine Lösung von 126,2 g (0,8 mol) an racemischem 1-(4-Chlor-phenyl)-ethylamin in 400 ml Dimethoxyethan wird bei Raumtemperatur unter Rühren nacheinander mit 98 g (0,8 mol) Chloressigsäureethylester und 6,2 g Novozym 435® (immobilisierte Lipase aus Candida antarctica; 7300 U/g) versetzt. Man rührt 3 Stunden und 15 Minuten bei Raumtemperatur und unterbricht dann die Reaktion, indem man das Enzym abfiltriert und mit 25 ml Dimethoxyethan nachwäscht.

### 2. Stufe

Das nach dem Abfiltrieren des Enzyms verbleibende Filtrat wird mit 250 ml Eiswasser und mit 68,5 ml (0,8 mol) konzentrierter wäßriger Salzsäure versetzt und danach unter vermindertem Druck (40-100 mbar) eingeengt. Man kühlt das Gemisch auf 5°C ab, filtriert den ausgefallenen Feststoff ab und wäscht mit 150 ml Eiswasser nach. Anschließend wird der farblose Feststoff auf Ton getrocknet. Man erhält auf diese Weise 85,5 g eines Produktes, das gemäß gaschromatographischer Analyse zu 99,85 % aus dem (R)-Enantiomeren des Chloressigsäure-1-(4-chlorphenyl)-ethylamids besteht. Der ee-Wert beträgt 99,1 %. Die Ausbeute errechnet sich zu 92,1 % der Theorie.
¹H-NMR-Spektrum (CDCl₃/TMS)
δ = 1,52 (d, 3H, CH₃); 4,05 (d, 2H, CH₂); 5,10 (m, 1H, CH); 7,24-7,37 (m, 4H, arom. Protonen) ppm

Die verbleibende wäßrige Phase wird zweimal mit je 100 ml Methylenchlorid extrahiert, dann unter Kühlung mit 100 ml konzentrierter, wäßriger Natronlauge versetzt und erneut mit Methylenchlorid extrahiert. Die vereinigten organischen Phasen werden über Natriumsulfat getrocknet und dann unter vermindertem Druck eingeengt. Man erhält auf diese Weise 58,7 g eines Produktes, das gemäß gaschromatographischer Analyse zu 93,2 % aus dem (S)-Enantiomeren des 1-(4-Chlorphenyl)-ethylamins besteht. Der ee-Wert beträgt 97,2 %. Die Ausbeute errechnet sich zu 88,1 % der Theorie.

### 3. Stufe

Eine Suspension aus 85,3 g des (R)-Enantiomeren des Chloressigsäure-1-(4-chlorphenyl)-ethylamids in 300 ml Wasser wird mit 94,5 ml konzentrierter, wäßriger Salzsäure versetzt und 18 Stunden unter Rückfluß erhitzt. Danach wird das Gemisch durch Zugabe von wäßriger Natronlauge alkalisch gestellt und mehrfach mit Methylenchlorid extrahiert. Die vereinigten organischen Phasen werden über Natriumsulfat getrocknet und dann unter vermindertem Druck eingeengt. Man erhält auf diese Weise 54,35 g eines Produktes, das gemäß gaschromatographischer Analyse zu 99,7 % aus dem (R)-Enantiomeren des 1-(4-Chlor-phenyl)-ethyl-amins besteht. Der ee-Wert beträgt 97,7 %. Die Ausbeute errechnet sich zu 87,4 % der Theorie.

Der Biokatalysator wurde in 6 weiteren identischen Versuchen eingesetzt. Dabei zeigte sich ein Aktivitätsverlust von 10 bis 15 %.

### Beispiel 4

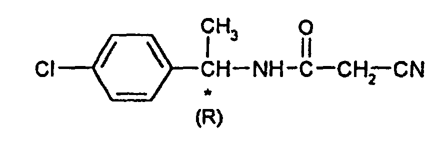

Eine Lösung von 6,5 g (0,04 mol) an racemischem 1-(4-Chlor-phenyl)-ethylamin in 30 ml 2-Cyan-essigsäure-ethylester wird bei Raumtemperatur mit 0,31 g Novozym 435® (= immobilisierte Lipase aus Candida antarctica; 7300 U/g) versetzt. Man rührt 3 Stunden bei 40°C und unterbricht dann die Reaktion, indem man das Enzym absaugt und mit 150 ml Methylenchlorid nachwäscht.

Das nach dem Abfiltrieren des Enzyms verbleibende Filtrat wird mit 50 ml verdünnter, wäßriger Salzsäure versetzt. Die organische Phase wird abgetrennt, über Natriumsulfat getrocknet und unter vermindertem Druck eingeengt. Man erhält auf diese Weise 3,76 g eines Produktes, das gemäß gaschromatographischer Analyse zu 98,5 % aus dem (R)-Enantiomeren des 2-Cyano-essigsäure-1-(4-chlorphenyl)-ethylamids besteht. Der ee-Wert beträgt 95,7 %. Die Ausbeute errechnet sich zu 83,4 % der Theorie.
¹H-NMR-Spektrum (CDCl₃/TMS)
δ = 1,52 (d, 3H, CH₃); 3,37 (s, 2H, CH₂); 5,07 (m, 1H, CH); 6,3 (s, 1H, NH); 7,23-7,35 (m, 4H, aromat. Protonen) ppm

### Beispiel 5

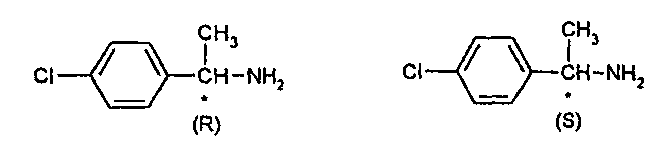

und

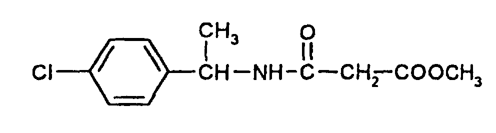

Ein Gemisch aus 6,2 g (0,04 mol) an racemischem 1-(4-Chlor-phenyl)-ethylamin, 0,3 g Novozym 435® (= immobilisierte Lipase aus Candida antarctica; 7300 U/g) und 65 ml Dimethoxyethan wird 5 Stunden bei 30°C gerührt. Danach unterbricht man die Reaktion, indem man das Enzym absaugt.

Das nach dem Absaugen des Enzyms verbleibende Filtrat wird mit 50 ml 10 %iger, wäßriger Salzsäure versetzt und dann unter vermindertem Druck eingeengt. Das anfallende Gemisch wird dreimal mit je 50 ml Methylenchlorid extrahiert und dann mit konzentrierter, wäßriger Natronlauge alkalisch gestellt. Man extrahiert die wäßrige Phase erneut mehrfach mit Methylenchlorid, trocknet die vereinigten organischen Phasen über Natriumsulfat und engt unter vermindertem Druck ein. Es verbleiben 2,9 g eines Produktes, das gemäß gaschromatographischer Analyse zu 95 % aus dem (S)-Enantiomeren des 1-(4-Chlor-phenyl)-ethylamins besteht. Der ee-Wert beträgt 72 %. Ausbeute: 44,8% der Theorie.

Die vor der Behandlung mit Natronlauge erhaltene Methylenchlorid-Lösung (erste Extraktion) wird unter vermindertem Druck eingeengt. Man erhält ein Produkt, das im wesentlichen aus dem (R)-Enantiomeren des Malonsäuremethylester-1-(4-chlorphenyl)-ethylamids besteht.
¹H-NMR-Spektrum (CDCl₃/TMS)
δ = 1,48 (d, 3H, CH₃); 3,35 (s, 2H, CH₂); 3,75 (s, 3H, CH₃); 5,1 (m, 1H, CH); 7,26-7,29 (m, 4H, aromat. Protonen) ppm.

Das zuvor erhaltene Produkt aus dem (R)-Enantiomeren des Malonsäuremethylester-1-(4-chlor-phenyl)-ethylamids wird mit 20 ml halbkonzentrierter, wäßriger Salzsäure versetzt und 9 Stunden unter Rückfluß erhitzt. Danach wird das Gemisch durch Zugabe von wäßriger Natronlauge alkalisch gestellt und mehrfach mit Methylenchlorid extrahiert. Die vereinigten organischen Phasen werden über Natriumsulfat getrocknet und dann unter vermindertem Druck eingeengt. Man erhält auf diese Weise 2,8 g eines Produktes, das gemäß gaschromatographischer Analyse zu 95 % aus dem (R)-Enantiomeren des 1-(4-Chlor-phenyl)-ethylamins besteht. Der ee-Wert beträgt 93 %. Die Ausbeute errechnet sich zu 42,8 % der Theorie.

## Patentansprüche

1. Verfahren zur Herstellung von optisch aktiven Aminen der Formel 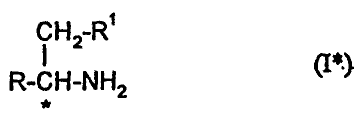 in welcher
R für einen Rest der Formel
-(CH₂)ₘ-R²
steht, worin
R² für gegebenenfalls substituiertes Phenyl der Formel 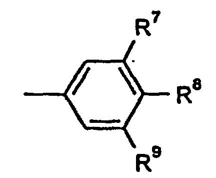 steht, wobei
R⁷, R⁸ und R⁹ unabhängig voneinander für Wasserstoff, Halogen, Alkyl mit 1 bis 4 Kohlenstoffatomen, Alkbxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Cyano, Dialkylamino mit 1 bis 4 Kohlenstoffatomen in jeder Alkylgruppe, Nitro, Phenyl, Phenoxy oder Benzyl stehen,
und
m für die Zahlen 0, 1, 2 oder 3 steht,
und
R¹ für Wasserstoff steht,
**dadurch gekennzeichnet, daß** man
a) in einer ersten Stufe racemische Amine der Formel 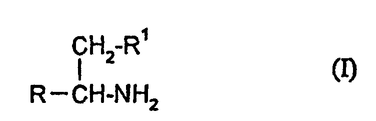 in welcher
R und R¹ die oben angegebenen Bedeutungen haben,
mit Estern der Formel 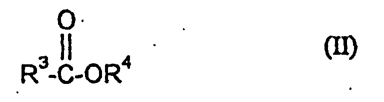 in welcher
R³ geradkettiges Halogenalkyl mit 1 bis 4 Kohlenstoffatomen und 1 bis 3 Fluor- und/oder Chloratomen oder für einen Rest der Formel -CH₂-C≡N steht,
oder
R³ für einen Rest der Formel -CH₂-COOR⁶ steht, worin
R⁶ für Methyl, Ethyl, n-PropyDoder n-Butyl steht,
und
R⁴ für geradkettiges Alkyl mit 1 bis 8 Kohlenstoffatomen steht, in Gegenwart von Lipase aus Candida antarctica und in Gegenwart eines Verdünnungsmittels umsetzt,
b) in einer zweiten Stufe das erhaltene Gemisch aus (S)-Amin der Formel 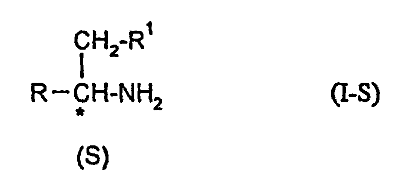 in welcher
R und R¹ die oben angegebenen Bedeutungen haben,
und acyliertem (R)-Amin der Formel 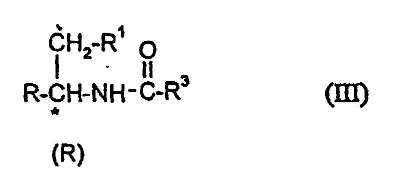 in welcher
R, R¹ und R³ die oben angegebenen Bedeutungen haben,
trennt und
c) gegebenenfalls in einer dritten Stufe aus acyliertem (R)-Amin der Formel (III) durch Behandlung mit Säure oder Base, gegebenenfalls in Gegenwart eines Verdünnungsmittels (R)-Amin der Formel 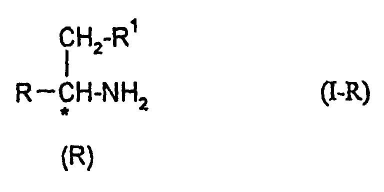 in welcher
R und R¹ die oben angegebenen Bedeutungen haben,
freisetzt.

2. Verfahren gemäß Anspruch 1, **dadurch gekennzeichnet, daß** man als racemisches Amin der Formel (I) das 1-(4-Chlorphenyl)-ethylamin der Formel 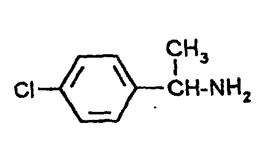 einsetzt.

3. Verfahren gemäß Anspruch 1, **dadurch gekennzeichnet, daß** man als Ester der Formel (II) den Chloressigsäure-ethylester der Formel 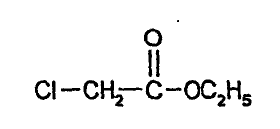 einsetzt.

4. Verfahren gemäß Anspruch 1, **dadurch gekennzeichnet, daß** man bei der Durchführung der ersten Stufe bei Temperaturen zwischen 0°C und 80°C arbeitet.

5. Acylierte (R)-Amine der Formel 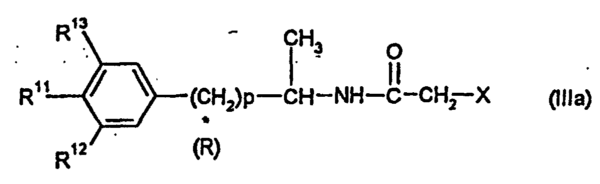 in welcher
R¹³ und R¹² für Methyl stehen,
R¹¹ für Wasserstoff steht,
p für die Zahl 2 steht und
X für Chlor oder Cyano steht,
oder
R¹³, R¹¹ und R¹² für Wasserstoff stehen,
p für die Zahlen 1 oder 2 steht und
X für Chlor oder Cyano steht,
oder
R¹¹ für Fluor, Chlor, Brom, Methyl, Methoxy oder Methylthio steht,
R¹³ und R¹² für Wasserstoff stehen,
p für die Zahl 0, 1 oder 2 steht und
X fiir Chlor oder Cyano steht.

## Claims

1. Process for preparing optically active amines of the formula 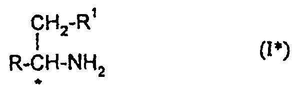 in which
R represents a radical of the formula
-(CH₂)ₘ-R²,
in which
R² represents optionally substituted phenyl of the formula 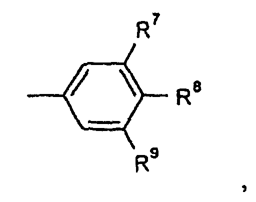 in which
R⁷, R⁸ and R⁹ independently of one another each represent hydrogen, halogen, alkyl having 1 to 4 carbon atoms, alkoxy having 1 to 4 carbon atoms, alkylthio having 1 to 4 carbon atoms, halogenoalkyl having 1 to 4 carbon atoms and 1 to 5 identical or different halogen atoms,halogenoalkoxy having 1 to 4 carbon atoms and 1 to 5 identical or different halogen atoms, cyano, dialkylamino having 1 to 4 carbon atoms in each alkyl group,nitro, phenyl, phenoxy or benzyl,
and
m represents the numbers 0, 1, 2 or 3,
and
R¹ represents hydrogen,
**characterized in that**
a) in a first step, the racemic amines of the formula 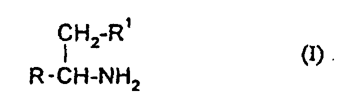 in which
R and R¹ are each as defined above
are reacted with esters of the formula 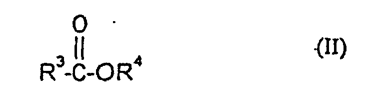 in which
R³ represents straight-chain halogenoalkyl having 1 to 4 carbon atoms and 1 to 3 fluorine and/or chlorine atoms, or represents a radical of the formula -CH₂-C≡N
or
R³ represents a radical of the formula -CH₂-COOR⁶, in which
R⁶ represents methyl, ethyl, n-propyl or n-butyl
and
R⁴ represents straight-chain alkyl having 1 to 8 carbon atoms,
in the presence of lipase from Candida antarctica and in the presence of a diluent,
b) separating, in a second step, the resulting mixture of (S)-amine of the formula 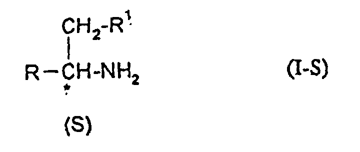 in which
R and R¹ are each as defined above,
and acylated (R)-amine of the formula 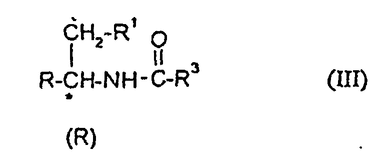 in which
R, R¹ and R³ are each as defined above,
is separated and
c) if appropriate, in a third step, the (R)-amine of the formula 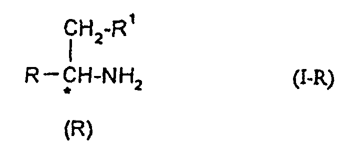 in which
R and R¹ are each as defined above,
is set free from acylated (R)-amine of the formula (III) by treatment with acid or base, if appropriate in the presence of a diluent.

2. Process according to Claim 1, **characterized in that** the racemic amine of the formula (I) used is 1-(4-chlorophenyl)-ethylamine of the formula

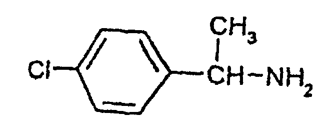

3. Process according to Claim 1, **characterized in that** the ester of the formula (II) used is ethyl chloroacetate of the formula

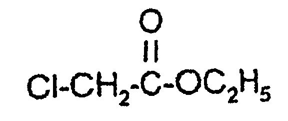

4. Process according to Claim 1, **characterized in that** the first step is carried out at temperatures between 0°C and 80°C.

5. Acrylated (R)-amines of the formula 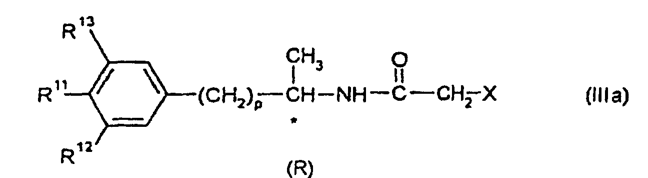 in which
R¹³ and R¹² each represent methyl,
R¹¹ represents hydrogen,
p represents the number 2 and
X represents chlorine or cyano,
or
R¹³, R¹¹ and R¹² each represent hydrogen,
p represents the numbers 1 or 2 and
X represents chlorine or cyano.
or
R¹¹ represents fluorine, chlorine, bromine, methyl, methoxy or methylthio,
R¹³ and R¹² each represent hydrogen,
p represents the number 0, 1 or 2 and
X represents chlorine or cyano.

## Revendications

1. Procédé de production d'amines optiquement actives de formule 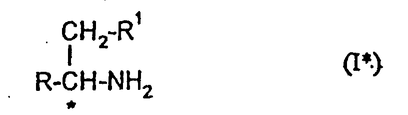 dans laquelle
R représente un reste de formule
- (CH₂)ₘ-R²,
où
R² est un reste phényle éventuellement substitué de formule 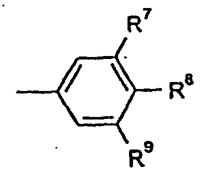 dans laquelle
R⁷, R⁸ et R⁹ représentent, indépendamment les uns des autres l'hydrogène, un halogène, un groupe alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes identiques ou différents, halogénalkoxy ayant 1 à 4 atomes de carbone et 1 à 5 atomes d'halogènes identiques ou différents, cyano, dialkylamino ayant 1 à 4 atomes de carbone dans chaque groupe alkyle, nitro, phényle, phénoxy ou benzyle,
et
m représente le nombre 0, 1, 2 ou 3,
et
R¹ est l'hydrogène,
**caractérisé en ce que** :
a) dans une première étape, on fait réagir des amines racémiques de formule 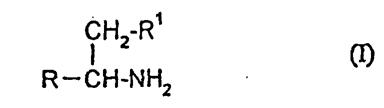 dans laquelle
R et R¹ ont les définitions ci-dessus,
avec des esters de formule 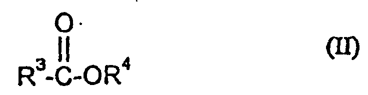 dans laquelle
R³ est un reste halogénalkyle linéaire ayant 1 à 4 atomes de carbone et 1 à 3 atomes de fluor et/ou de chlore, ou un reste de formule -CH₂-C≡N,
ou bien
R³ représente un reste de formule -CH₂-COOR⁶, dans laquelle
R⁶ est un radical méthyle, éthyle, n-propyle ou n-butyle,
et
R⁴ est un groupe alkyle linéaire ayant 1 à 8 atomes de carbone,
en présence de lipase extraite de Candida antarctica et en présence d'un diluant,
b) dans une deuxième étape, on sépare le mélange obtenu formé de (S)-amine de formule 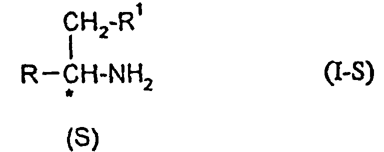 dans laquelle
R et R¹ ont les définitions indiquées ci-dessus et de (R)-amine acylée de formule 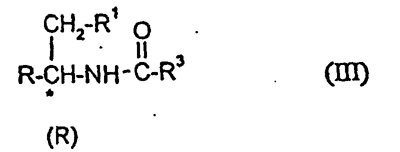 dans laquelle
R, R¹ et R³ ont les définitions ci-dessus,
et
c) le cas échéant, dans une troisième étape, on libère de la (R)-amine acylée de formule (III), par traitement avec un acide ou une base, éventuellement en présence d'un diluant, la (R)-amine de formule 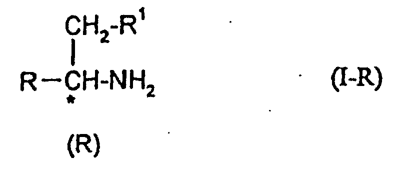
dans laquelle
R et R¹ ont les définitions indiquées ci-dessus.

2. Procédé suivant la revendication 1, **caractérisé en ce qu'**on utilise comme amine racémique de formule (I) la 1-(4-chlorophényl)-éthylamine de formule

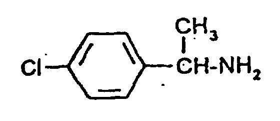

3. Procédé suivant la revendication 1, **caractérisé en ce qu'**on utilise comme ester de formule (II) l'ester éthylique d'acide chloroformique de formule

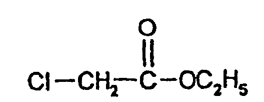

4. Procédé suivant la revendication 1, **caractérisé en ce qu'**on opère à des températures comprises entre 0°C et 80°C dans la conduite de la première étape.

5. (R)-amines acylées de formule 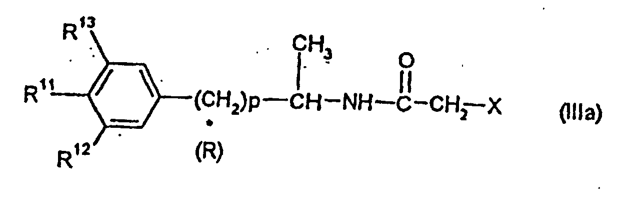 dans laquelle
R¹³ et R¹² sont des groupes méthyle,
R¹¹ est l'hydrogène,
p est le nombre 2 et
X représente le chlore ou le groupe cyano,
ou bien
R¹³, R¹¹ et R¹² représentent l'hydrogène,
p représente le nombre 1 ou 2 et
X est le chlore ou un groupe cyano,
ou bien
R¹¹ est le fluor, le chlore, le brome, un groupe méthyle, méthoxy ou méthylthio,
R¹³ et R¹² représentent l'hydrogène,
p représente le nombre 0, 1 ou 2 et
X est le chlore ou le groupe cyano.
